(19) Europäisches Patentamt
European Patent Office
Office européen des brevets

(11) EP 4 083 053 A1

(12) EUROPEAN PATENT APPLICATION

(43) Date of publication:
**02.11.2022 Bulletin 2022/44**

(21) Application number: **21171717.8**

(22) Date of filing: **30.04.2021**

(51) International Patent Classification (IPC):
***C07K 7/06*** *(2006.01)* ***C07K 1/14*** *(2006.01)*
***B01D 15/18*** *(2006.01)*

(52) Cooperative Patent Classification (CPC):
**C07K 7/06; B01D 15/1807; B01D 15/1871; B01D 15/325**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **Fresenius Kabi iPSUM S.r.l.**
  **20051 Cassina de' Pecchi - Milano (IT)**
- **Università Degli Studi Di Ferrara**
  **44121 Ferrara (IT)**

(72) Inventors:
- **RICCI, Antonio**
  **20060 Cassina de' Pecchi (IT)**
- **MACIS, Marco**
  **20060 Cassina de' Pecchi (IT)**
- **CAVAZZINI, Alberto**
  **44121 Ferrara (IT)**
- **FELLETTI, Simona**
  **44121 Ferrara (IT)**
- **CABRI, Walter**
  **40138 Bologna (IT)**
- **CATANI, Martina**
  **44121 Ferrara (IT)**
- **DE LUCA, Chiara**
  **44121 Ferrara (IT)**

(74) Representative: **Fresenius Kabi Deutschland GmbH**
**Patent Department**
**Pharmaceuticals Division**
**Borkenberg 14**
**61440 Oberursel (DE)**

(54) **METHOD FOR ICATIBANT PREPARATION**

(57) The present invention provides a method for the preparation of icatibant comprising a chromatographic process for removing impurities from a solution containing icatibant with a purity below specification, wherein fractions corresponding to overlapping regions, containing both icatibant and impurities, are recycled and undergo further purification together with amounts of the original feed solution in an iterative process.

Fig. 1

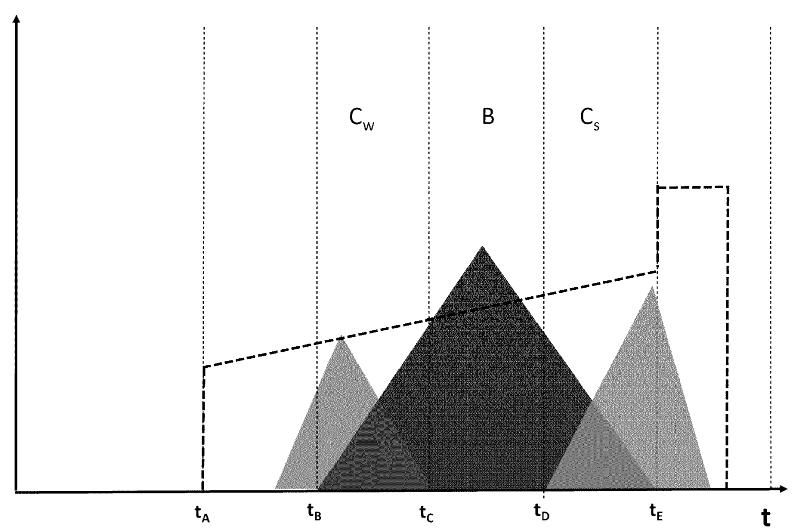


EP 4 083 053 A1

## Description

### Field of the invention

The invention relates to the field of peptide preparation. In particular, it relates to a method for the preparation of icatibant comprising a process for removing impurities by reversed-phase chromatography.

### Background art

Icatibant (CAS No. 130308-48-4) is a small peptide of ten amino acids, indicated as H-D-Arg-Arg-Pro-Hyp-Gly-Thia-Ser-D-Tic-Oic-Arg-OH, which may be synthesized through solid phase peptide synthesis (SPPS). Icatibant is a selective and specific antagonist of bradykinin B2 receptors commonly used in the treatment of symptoms of hereditary angioedema, and has the following chemical formula (1):

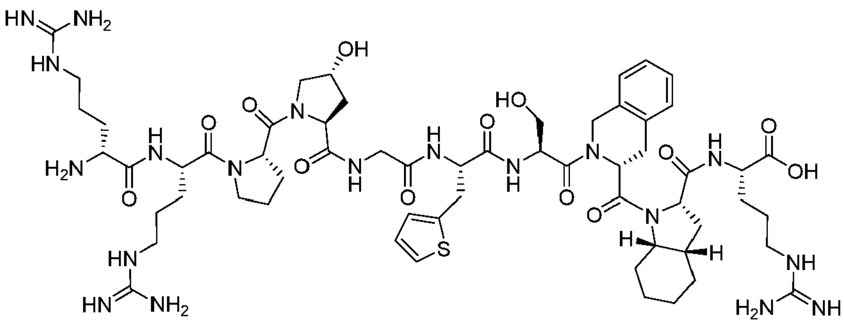

1

The purification of icatibant through reversed-phase chromatography was described in several patent applications, e.g. in CN104072585. However, such purification is challenging because product-related impurities, i.e. compounds produced during the synthesis with very similar chemical characteristics to the target compound, often show a chromatographic behaviour very similar to the target. In this situation a chromatogram shows overlapping regions where target product and impurities are co-eluted. On the one hand, collecting those fractions corresponding to these overlapping regions improves the yield of the separation process at the expense of the overall purity. On the other hand, discharging said fractions preserves the overall purity, which is mandatory for a product being the active ingredient of a pharmaceutical product, at the expense of the process yield.

In addition, reversed-phase chromatography requires large volumes of solvents, creating challenges in disposing or recycling such solvents in an eco-compatible manner.

There is therefore a need for a purification method capable of increasing the icatibant recovery and the overall process productivity while maintaining the icatibant purity, and which is suitable for industrial application.

The present invention has the purpose of solving the above problems or at least improving product recovery and process productivity.

### Summary of the invention

It has been surprisingly found that higher product recovery and process productivity in the preparation of pure icatibant can be achieved by applying a chromatographic process wherein fractions corresponding to overlapping regions in a chromatogram, containing both icatibant and impurities, are recycled and undergo further purification together with amounts of the original feed solution.

Accordingly, the present invention provides a method for the preparation of icatibant comprising a chromatographic process for removing impurities from a solution A containing icatibant with a purity below specification, comprising the steps of:

a) providing n +1 amounts of the solution A;

b) subjecting an amount a of solution A to a first reversed phase HPLC purification run, wherein the stationary phase is hydrocarbon bonded silica, the mobile phase comprises an aqueous buffer and an organic modifier, and a gradient elution is effected by gradually increasing the organic modifier concentration within the mobile phase over time; said

reversed phase HPLC purification run comprising loading a chromatographic column with the amount a of solution A; and wherein one or more fractions B containing icatibant with a purity above specification and one or more fractions C containing icatibant with a purity below specification are collected;

c) pooling the fractions C;

d) subjecting the pooled fractions C obtained in step c) together with an amount b of solution A to a further reversed phase HPLC purification run as described in step b); said further reversed phase HPLC purification run comprising loading a chromatographic column with said pooled fractions C and with the amount b of solution A;

e) optionally repeating step c) and d) until the n amounts b of solution A have been subjected to at least one reversed phase HPLC purification run as described in step d); and

f) pooling all fractions B;

wherein n is at least 1.

As a result, the overall recovery of icatibant with a purity above specification which is obtained by the method of the invention, for instance higher than 99% (by HPLC), is unexpectedly and remarkably higher than what can be obtained by a single purification run, by up to at least around sevenfold. The process productivity can be also increased by up to at least around four- to sevenfold.

## Drawings

Fig. 1: Schematic representation of a batch chromatogram, wherein overlapping elution regions containing icatibant (B) and icatibant plus impurities (C) are illustrated. The dashed line represents the gradient elution with increasing amount of organic modifier over time (t). The time points $t_A$ to $t_E$ are indicated at the X-axis.

Fig. 2: Schematic representation of half a cycle (i.e a "switch") in Two-column Countercurrent Solvent Gradient Purification and how it corresponds with its batch chromatogram. The two columns are indicated as C1 and C2. The different tasks of each column are indicated with the number in the circle. W refers to Waste. The dashed line represents the gradient elution with increasing amount of organic modifier over time (t). Where a line is connecting the two columns it indicates that the columns are connected during those tasks (2→6, 4→8), to allow the eluate of column C1 to load onto column C2. The time points tA to tE are indicated at the X-axis.

Fig. 3: Schematic representation of four time windows (I=$T_W$, II=$T_B$, III=$T_S$) defining a switch in a Two-column Countercurrent Solvent Gradient Purification. The two columns indicated as C1 and C2 are alternatingly connected and disconnected. The different tasks of each column are indicated with the number in the circle. W refers to Waste. ID refers to inline dilution.

## Detailed Description of the invention

"Chromatography" or "chromatographic process" is a well-known physical method of separation that distributes components to separate between two phases, one stationary (stationary phase), the other (the mobile phase) moving in a definite direction such as to obtain the separation. HPLC is an example of chromatography, in particular referring to High Performance Liquid Chromatography. The process of the mobile phase moving through the stationary phase is also called "elution". Accordingly, the mobile phase is sometimes referred to as "eluent" when referring to its movement through the column, and as "eluate" when referring to its exit from the column. During the elution, set volumetric amounts of mobile phase, i.e. fractions, are collected which contain the desired component, herein the peptide icatibant. For the purpose of the invention, the "chromatographic process" refers to the whole method of the invention, which involves more than one chromatographic run.

The term "chromatography column" or "column" as used herein refers to a tube filled with the stationary phase through which the mobile phase moves, from head to tail. Such columns are also referred to as HPLC preparative columns.

The term "column volume" (or CV) as used herein refers to the geometric volume inside of a column and it is measured in mL. This volume includes both the volume occupied by the stationary phase and the volume available for the mobile phase movement.

The term "reversed phase HPLC purification" (also referred to as RP-HPLC purification) as used herein refers

to a purification which comprises at least one step of High Performance Liquid Chromatography wherein the mobile phase is significantly more polar than the stationary phase.

In RP-HPLC purification, the stationary phase is typically a non-polar solid hydrophobic stationary phase consisting of hydrocarbons or strongly hydrophobic polymers of varying lengths, such as for instance hydrocarbon bonded silica.

In RP-HPLC purification, the mobile phase, or eluent, is generally an aqueous composition comprising a buffer and an organic modifier, which is an organic solvent. The composition of the mobile phase may vary during the chromatography over time by mixing different aqueous buffers with different amounts of organic modifier.

The term "gradient elution" as used herein refers to an elution which is carried out by gradually increasing the organic modifier concentration within the mobile phase during the chromatographic run.

The term "RP-HPLC chromatographic run" or "purification run" as used herein refers to one whole passage through the chromatography column of an amount of the feed solution or of fractions C or both, and comprises the following four or five consecutive steps: equilibrating, loading, optionally washing, gradient elution and stripping. The changing in composition of the mobile phase over time during the gradient elution is referred to as "gradient slope". The washing step may be present or absent.

The term "equilibrating step" or "equilibrating" as used herein refers to an elution step wherein a mobile phase is eluted through the chromatography column which has the initial composition according to the gradient, typically with the lowest amount of organic modifier.

The term "loading" or "loading step" as used herein refers to a step of introducing an amount of solution A or fractions C (such as fractions Cw or fractions $C_S$, as defined below) or mixtures thereof onto the head of a chromatography column during the process according to the invention.

The term "washing step" as used herein refers to an elution step, generally occurring after the loading, wherein a mobile phase is eluted through the column which has a composition according to the initial gradient, and which step is meant to remove salts that may be present in the feed solution, i.e. the solution which contains the components to be separated. Alternatively, the washing step may be performed with a mobile phase the composition of which differs from the composition according to the initial gradient and is followed by a further equilibrating step.

The term "gradient (eluting) step" as used herein refers to an elution step occurring after the washing step or the loading step wherein a mobile phase is eluted through the chromatography column changing its composition over time according to the set gradient slope.

The term "stripping step" as used herein refers to the final elution step wherein a mobile phase is eluted through the chromatography column which has a composition suitable to remove contaminants and regenerate the column to its original condition, typically having the highest amount of organic modifier.

The amount of mobile phase used in every step may be expressed in column volumes, CVs, thus being independent on the chromatographic column used.

The term "batch run" as used herein refers to a single chromatographic run, which is performed for the purpose of setting up the parameters of the method according to the invention, e.g. the gradient slope, or for the purpose of performance comparison. The batch run allows the identification of a "batch chromatogram", which is a chromatogram showing icatibant, its impurities and the overlapping regions, where icatibant and impurities are co-eluted.

The term "solution A" or "feed solution" is the solution containing icatibant and its impurities which undergoes reversed phase HPLC purification in the process according to the invention.

Amounts of solution A are volumetric amounts, usually measured in mL. The "amount a of solution A" refers to the initial volume of solution A which is subjected to step b) in the method of the invention, while the "amount b of solution A" refers to the volume of solution A which is subjected to each subsequent step d) of said method.

The term "fraction pooling" as used herein refers to a step of collecting fractions obtained during the elution. Fractions B are defined as fractions containing icatibant with a purity above specification. Fractions C are defined as fractions containing icatibant with a purity below specification, also referred to as "recycling fractions".

The term "impurity" as used herein refers to an unwanted substance, co-solved in the solution A, which differs from icatibant and thereby lowers the purity of icatibant. The impurity has to be removed in order to use icatibant, for instance as an active pharmaceutical ingredient.

The term "weak impurity" as used herein refers to an impurity which is more weakly adsorbed onto the stationary phase than icatibant and therefore elutes earlier than icatibant in a chromatographic run used for purification of icatibant as described herein.

The term "strong impurity" as used herein refers to an impurity which is more strongly adsorbed onto the stationary phase than icatibant and therefore elutes later than icatibant in a chromatographic run used for purification of icatibant as described herein.

"Purity" (%) as used herein is measured by HPLC and is calculated as the % ratio between the chromatographic area (A) under the icatibant peak (A product) and the sum of the areas under all the peaks (A total) in the HPLC chromatogram:

$$\text{Purity (\%)} = \frac{\text{A product}}{\text{A total}} \times 100$$

The term "specification" as used herein refers to the desired purity (%) value, i.e. the purity which is obtained after having performed the method according to the invention. This value is set. It could be, for example the purity value required for a certain regulatory approval process. Therefore, "Purity above specification" means purity (%) which is equal or higher than the set purity specification value.

The term "crude icatibant" or "crude" refers to icatibant in a solid or liquid form, i.e. for instance a solution comprising icatibant, having purity below specification, regardless of the origin of such icatibant, which may be any synthetic process or any purification process or a combination of both.

"Recovery" (%) is calculated for the overall process according to the invention as the % ratio between the final amount of icatibant obtained with purity above specification and the total quantity of icatibant loaded as solution A during the chromatographic process:

$$\text{Recovery (\%)} = \frac{\text{m obtained}}{\text{m loaded}} \times 100$$

As such, the recovery is independent from the concentration of icatibant in the crude or in the feed solution.

Purity and Recovery values are given in %.

In particular, purity and recovery may also be calculated for single collected fractions. The calculation may be made by sampling directly a specific amount of the solution and applying the set analytical method, as described in Example 2. The recovery of a fraction is the absolute quantity of icatibant in the fraction, which can be calculated through comparison with a calibration curve.

"Productivity" (mg/mL/h) is calculated for the overall process as the final amount (mg) of icatibant obtained with purity above specification per unit time (h) and column volume (mL):

$$\text{Productivity} = \frac{\text{m obtained}}{\text{CV} \times \text{operation time}}$$

Wherein the operation time is the duration of the whole process according to the invention, which is the sum of all the steps performed, and wherein the CV refers to the single column or to the sum of all the single CV when more than one column is employed in the process. Thus this parameter indicates how much product is produced per minute and per CV.

"Solvent Consumption" (L/g) is calculated as the volume of mobile phase (L) used per unit amount (g) of icatibant obtained with purity above specification:

$$\text{Solvent Consumption (L/g)} = \frac{V_{MP}}{\text{m obtained}}$$

The term "inline dilution" as used herein refers to the automatic dilution which may occur when loading the recycling fractions C onto the column in particular embodiments of the process of the invention, namely when more than one single column is used to perform steps d) according to the invention. The dilution is carried out with a mobile phase containing no or a low amount of organic modifier, in order to allow the adsorption of icatibant onto the column and prevent it from eluting too fast. In embodiments when one single column is used to perform the whole process, fractions C may be diluted manually before being loaded onto the column in each step d).

A common way to remove unwanted impurities from a crude icatibant is that of subjecting such sample to a single chromatographic run purification, typically involving a column, and collecting fractions with purity above specification, i.e. the desired purity, while discarding fractions which do not comply with that requirement. This kind of process usually leads to low recovery of icatibant, making the overall preparation of icatibant quite inefficient and expensive.

The present invention provides a method wherein only a partial amount of the crude icatibant, generally in the form of a feed solution, is subjected to the chromatographic run and the fractions with icatibant purity below specification are not discarded. These fractions are instead recycled and loaded onto the column in a subsequent chromatographic run, wherein they are further processed together with a further amount of the original icatibant feed solution. This results in an iterative process wherein the steps are repeated until the whole original amount of icatibant crude is purified.

Accordingly, the present invention provides a method for the preparation of icatibant comprising a chromatographic process for removing impurities from a solution A containing icatibant with a purity below specification, comprising the steps of:

a) providing n +1 amounts of the solution A;

b) subjecting an amount a of solution A to a first reversed phase HPLC purification run, wherein the stationary phase is hydrocarbon bonded silica, the mobile phase comprises an aqueous buffer and an organic modifier, and a gradient elution is effected by gradually increasing the organic modifier concentration within the mobile phase over time; said reversed phase HPLC purification run comprising loading a chromatographic column with the amount **a** of solution A; and wherein one or more fractions B containing icatibant with a purity above specification and one or more fractions C containing icatibant with a purity below specification are collected;

c) pooling the fractions C;

d) subjecting the pooled fractions C obtained in step c) together with an amount **b** of solution A to a further reversed phase HPLC purification run as described in step b); said further reversed phase HPLC purification run comprising loading a chromatographic column with said pooled fractions C and with the amount **b** of solution A;

e) optionally repeating step c) and d) until the n amounts **b** of solution A have been subjected to at least one reversed phase HPLC purification run as described in step d); and

f) pooling all fractions B;

wherein n is at least 1.

Preferably, n is higher than 1, more preferably n is higher than 2, most preferably n is higher than 4.

A solution A may be obtained by dissolving a crude icatibant which is the result of a synthetic process, for instance a preparation in solid phase (SPPS) or in liquid phase (LPPS) or a combination of the two, as described for instance in WO 2019202057. The crude icatibant that may be subjected to the chromatographic process of present invention may also be the result of any other preparation process, such as a precipitation, crystallization, centrifugation, or any other chromatographic purification, or any other purification step, which didn't result in a crude icatibant with a purity above specification. Accordingly, the crude icatibant may be characterized by any degree of purity below specification.

For the purpose of the present invention, the icatibant specification preferably is 95, 95.5, 96, 96.5, 97, 97.5, 98, 98.5, 99 or 99.5%, more preferably 98.5%, and most preferably 99%.

The solution A can be prepared by dissolving a crude icatibant in the solid form in a liquid composition comprising an aqueous buffer and an organic solvent.

The aqueous buffer may comprise an acid selected from the group consisting of phosphoric acid, trifluoroacetic acid, acetic acid, methanesulphonic acid and/or a base selected from the group consisting of ammonia, triethylamine, sodium hydroxide, potassium hydroxide. Preferably, the aqueous buffer is an ammonium acetate buffer.

The aqueous buffer concentration may range from 1 to 100 mM. Preferably the aqueous buffer concentration is from 20 to 50 mM. More preferably the aqueous buffer concentration is 50 mM.

The organic solvent may be selected among suitable solvents like acetonitrile, methanol, ethanol, and the like, preferably the organic solvent is acetonitrile. The amount of the organic solvent in the solution A may range approximately from 1 to 30% by volume. Preferably, the amount of the organic solvent in the solution A ranges from 2 to 10%, most preferably from 3 to 5 %.

The concentration of icatibant in the solution A may range from 0.1 to 50 mg/mL, preferably from 1 to 10 mg/mL, calculated on the effective concentration (% by weight) of icatibant in the crude, which can be determined by HPLC analysis by using a calibration curve, as well known to the person skilled in the art and as described in the Examples. Preferably, the concentration of icatibant in the solution A is from 1 to 10 mg/mL.

Alternatively, the solution A can be prepared by diluting or concentrating a solution of crude icatibant such as to obtain a solution A comprising an aqueous buffer and an organic solvent, with the composition and the concentration as defined above.

The pH of solution A may be optionally adjusted to the range 5.5-7.5 by addition of a diluted base, such as, for instance a 10% ammonium hydroxide solution.

The solution A is generally filtered before loading onto the chromatographic column in step b) or step d) of the method of the invention.

In the method according to the invention, the stationary phase used in the chromatographic process is selected

from the group consisting of C18, C8, C4, phenyl-hexyl, biphenyl, fluorophenyl and phenyl bonded silica, preferably it is C18 bonded silica, wherein C18 is octyldecylsilane and contains 18 carbons bound to the silica.

Such stationary phase may have particle size varying in the range from 5 to 50 micron and the like, for instance being 5, 6, 7, 8, 9, 10, 15, 20, 30, or 50 micron. The stationary phase pore size may vary in the range 60 to 300 angstrom, for instance being 60, 80, 90, 100, 125, 130, 200, 250, or 300 angstrom.

In the method of present invention, the mobile phase comprises an aqueous buffer and an organic modifier.

The organic modifier is an organic solvent whose concentration is increased in the mobile phase during the gradient elution. According to the invention, the organic modifier is selected from the group consisting of acetonitrile, methanol, ethanol and tetrahydrofuran, preferably it is acetonitrile.

The aqueous buffer suitable for the method of the present invention can be obtained by mixing acids, bases and/or salts in water at the desired concentration.

Accordingly, in the method of the present invention the aqueous buffer comprises an acid, selected from the group consisting of phosphoric acid, hydrochloric acid, trifluoroacetic acid, acetic acid, methanesulphonic acid, formic acid and citric acid; and/or a base, selected from the group consisting of ammonia, triethylamine, diethylamine, dibutylamine, diisopropylethylamine, sodium hydroxide and potassium hydroxide; and/or a salt, selected from the group consisting of formates, like sodium, ammonium and potassium formate; acetates, like sodium, ammonium and potassium acetate; phosphates, like sodium, potassium and triethylammonium phosphate; chlorides, like sodium and ammonium chloride; ammonium carbonate, ammonium bicarbonate and the like. Most preferably, the aqueous buffer comprises triethylammonium phosphate (TEAP).

The aqueous buffer may have a concentration from 0.05 mM to 1 M. In the method according to the invention the preferred concentration is preferably from 0.1 to 250 mM, more preferably from 10 to 100 mM, most preferably from 20 to 50 mM.

In a preferred embodiment the aqueous buffer is TEAP at a concentration of 20 mM.

Typically, the mobile phase entering the chromatographic column results from mixing two or more mobile phases at different compositions as to the amount of aqueous buffer and of organic modifier. For instance, two mobile phases, MP-A and MP-B, are mixed together during the elution such that the organic modifier concentration is gradually increased within the mobile phase over time. In the event that MP-A has the lowest amount of organic modifier, the amount of MP-B may be increased in the mobile phase during the gradient elution.

The amount of the organic modifier in the mobile phase may vary during the elution depending on the specific step performed.

The RP-HPLC chromatographic run generally comprises the steps of equilibrating, loading, washing, gradient elution and stripping. The washing step is optional.

The flow rates employed can be different or identical for the different steps, and they depend on the column volume. With the columns used in the Examples herein, the flow rate can vary in the range 0.1-10 mL/min. The flow rate may be easily adapted to the column size chosen.

Suitable preparative HPLC columns for the application of the method of the present invention may have, for instance, lengths varying from 100 to 700 mm or more, e.g. 100, 150, 200, or 250 mm, or the like; these columns may have for instance, diameters varying from 4 to 600 mm or more, e.g. 4, 6, 5, 10, 19, 20, 30, 50 or 100 mm, or the like. Bigger columns may also be used and operating conditions may be adapted by the skilled person.

The equilibrating step may be performed by eluting with an amount of organic modifier in the range 0 to 20% in the mobile phase.

The amount a of solution A to be loaded in step b) of the method of the invention is defined to comprise between 1 and 400 mg icatibant per CV, preferably between 10 and 50 mg/mLcolumn, more preferably about 10 $mg/mL_{column}$, wherein the quantity of icatibant considered is the effective quantity present in the crude which is used to prepare the solution A.

If a washing step is performed after loading, it may be performed by elution with an amount of organic modifier in the range 0 to 20% in the mobile phase.

During the subsequent gradient elution, the amount of organic modifier may vary in the range from 0 to 100% over a duration of 10 to 240 minutes. Fractions are collected periodically during the elution, and may be sampled, optionally diluted, and analyzed offline by HPLC to determine their purity and, optionally, to determine the quantity of icatibant contained in the single fraction (i.e. the recovery value). Analysis is performed by UV detection at preferred wavelengths of 220, 226, 265, 280 and 300 nm.

The gradient slope may be selected such as to obtain a reasonable separation, corresponding to a batch chromatogram like the one shown in Fig. 1, wherein a time window $T_B$ is defined (from tC to tD) wherein fractions B, containing icatibant with a purity above specification, are eluted.

Finally, a stripping step is performed, preferably by elution with an amount of organic modifier in the range 50 to 100% in the mobile phase.

Based on the analysis of the fractions collected during the chromatographic run of step b), at least two types

of fractions are determined: those containing icatibant with a purity above specification (fractions B) and at least one recycling fraction containing icatibant with a purity below specification (fractions C). Fractions B are pooled and put aside, optionally diluting them for storage purposes with the aqueous buffer used in the mobile phase of step b).

The fractions C are pooled in step c) of the method of the invention to continue the chromatographic process in step d).

Fractions C comprise fractions comprising icatibant and weak impurities, herein referred to as fractions Cw, or fractions comprising icatibant and strong impurities, herein referred to as fractions Cs, or both. Fractions $C_w$ are eluted earlier than fractions B while fractions Cs are eluted after fractions B.

In order to proceed with step d) of the method, the column which will be used in step d) undergoes the equilibrating step, performed as described above by elution with an organic modifier in the range 0 to 20% in the mobile phase, preferably with the same amount of organic modifier as used in step b). The column may be the same or another identical column as used in step b).

Next step is performed by loading the equilibrated chromatographic column with the pooled fractions C, which may be the sum of fractions $C_w$ and fractions $C_s$, and an amount b of solution A. Fractions C may be diluted with a mobile phase containing only an aqueous buffer or a low amount of organic modifier. For instance, MP-A may be used or the aqueous buffer used to prepare MP-A and/or MP-B.

Two equivalent loading modes are possible for said loading step in step d). In one embodiment, fractions C and the amount b are mixed and loaded simultaneously, and in a second embodiment they are loaded consecutively.

Regardless of the selected loading mode, the amount b has to be calculated such that the total quantity of icatibant by weight which is loaded in step d) is approximately the same as the quantity of icatibant by weight which had been loaded in step b) of the process. For the calculation, at least one of the values, either the recovery value of the pooled fractions B obtained in step b) or the recovery value of the pooled fractions C obtained in step c) has to be determined by HPLC as described above and in Example 2. The quantity of icatibant by weight which has to be loaded onto the column through the amount b of solution A is then calculated by difference with respect to the quantity of icatibant by weight which was loaded in step b) in the amount a of solution A. In other words, the quantity of icatibant in the amount b of solution A of step d) is not higher than the quantity of icatibant in the fractions B obtained in step b).

Step d) is then performed by using the same elution steps as in step b), i.e. the same mobile phase, same stationary phase, and the same gradient slope throughout the purification run.

Also during step d) elution, fractions are collected periodically and may be analyzed offline by HPLC as described above to determine their purity and optionally their recovery. Like in step b) described above, fractions B and fractions C are identified and separately pooled, and the chromatographic process continues iteratively until the whole solution A has been subjected to at least one RP-HPLC purification run as described in step d).

After one or two steps d), a steady-state is reached, meaning that the UV profiles of each subsequent step d) are completely superimposable step after step. Also, at steady-state, purity and recovery don't change anymore step after step. Therefore, the number of steps d) to be performed for the completion of the method according to the invention depends only on the amount of solution A that has to be processed.

In one embodiment, the method according to the invention is thus characterized by reversed phase HPLC purification runs of step b) and steps d) being performed with one chromatographic column. Said embodiment may be implemented as a manually operating method. This embodiment is exemplified in Example 5.

In the embodiment of the method of the invention as just described, in the reversed phase HPLC purification run of at least one of step d) either the fractions Cw and Cs are pooled and loaded onto the chromatographic column before or after loading the amount b of solution A; or the loading step is performed by loading first the fractions Cw, then the amount b of solution A and finally the fractions Cs.

The method of the invention is applicable also in case only fractions of type Cs or Cw are present, simply by omitting the specific loading step.

In such same embodiment, the reversed phase HPLC purification run of any step d) is performed on the same chromatographic column as the purification run of step b), after said column has been subjected to a stripping step and an equilibrating step between step b) and step d), and optionally before any subsequent step d).

In an alternative embodiment, the method according to the invention is characterized by reversed phase HPLC purification runs of step b) and steps d) being performed with a system of two or more identical interconnected chromatographic columns working alternatingly in disconnected or interconnected mode. This means that the columns employed in the method work in series (= interconnected: the mobile phase eluting from one column is loaded directly onto the next column) or in parallel (= disconnected, as in a single column chromatographic run: the mobile phase eluting from one column is collected in fractions or is discarded in the waste (W)), depending on the type of fractions eluting.

In other words, the two or more identical interconnected chromatographic columns can be connected to allow eluting fractions C to be subjected to another reversed phase HPLC purification run of step d); or disconnected to allow the eluate to be discarded or fractions B to be collected.

The respective connection/disconnection between the columns during the elution is regulated by tubing and

the movement of inlet and outlet column valves. In a preferred embodiment, this method is performed with a system of two identical interconnected chromatographic columns, column 1 and column 2. Said preferred embodiment may be implemented as an automatedly operating method. Such a method is also referred to as Two-column Countercurrent Solvent Gradient Purification (see also De Luca et al. in Trends in Analytical Chemistry 132 (2020) 116051).

Prior to setting up the method of such an embodiment, a batch run is performed, defining a batch chromatogram, schematically represented in Fig.1. Five time points are determined during the performance of such batch run, referred to as "switching times", which are the key design parameters. The Two-column Countercurrent Solvent Gradient Purification is exemplified in Example 6.

In the batch run, an amount **a** of solution A is subjected to a chromatographic run as defined above and the collected fractions are analyzed. Fractions B and recycling fractions C are identified; in particular, fractions $C_w$ and fractions Cs are identified, if present. The five switching times to be determined are: tA, which is when the gradient elution starts; tB, which is when icatibant starts eluting, mixed with weak impurities; tC, which is when icatibant starts eluting with purity above specification; tD, which is when icatibant is still eluting but together with strong impurities; and tE, which is when icatibant stops eluting from the column. The switching times are then used to set up the method of the invention employing a system of two identical columns, which is exemplified in Example 6.

During step b) of the method of the invention according to this embodiment, which is performed onto column 1, tB and tC are the time points that define the time window Tw for the elution of fractions Cw; tC and tD are the time points that define the time window $T_B$ for the elution of fractions B; tD and tE are the time points that define the time window $T_S$ for the elution of fractions Cs.

During the time window Tw and during the time window Ts, column 1 is connected to column 2 and the respective recycling fractions Cw and Cs eluting from column 1 are loaded onto column 2. During these steps, the respective fractions C are diluted inline with a mobile phase containing a low amount of organic modifier in order to allow the adsorption onto column 2 and prevent a too fast elution through column 2 of icatibant. Preferably, the mobile phase may be used which has the lowest amount of organic modifier, e.g. MP-A. In particular, the Cw fractions are diluted until the organic modifier concentration corresponding to the value it has at tB is reached, so that only icatibant adsorbs. On the other hand, the Cs fractions are diluted until the organic modifier concentration corresponding to the value it has at tA is reached, so that both icatibant and the strong impurities are adsorbed.

During the time window $t_B$ column 1 is disconnected from column 2 thereby allowing the elution and collection of fractions B.

tC and tD are the time points when the inlet and outlet column valves of the system switch their position.

At tC the valves position switches such that the fractions B eluting from column 1 are collected. At tD the valves position switches again such that fractions $C_w$ are loaded onto column 2.

Accordingly, each column has to perform 8 tasks. These tasks define one cycle. They can be described as follows:

1. elution of weak impurities, going to the waste (W)
2. elution of fractions $C_w$
3. elution of fractions B (collected)
4. elution of fractions $C_s$
5. stripping and equilibrating, wherein strong impurities go to the waste
6. loading of fractions $C_w$ eluting from the other column
7. loading of an amount **b** of solution A
8. loading of fractions $C_s$ eluting from the other column.

This means that the order of the tasks performed by one column is shifted by half a cycle with respect to the other: for instance, when column 1 is undergoing task 2, the column 2 is undergoing task 6, etc. When the columns come back to the initial positions, a cycle is completed. Half a cycle, also referred to as a "switch", is schematically represented in Fig. 2.

During tasks 2, 4, 6 and 8 the columns are connected, the system works in interconnected mode. During tasks 1, 3, 5 and 7 the columns are disconnected, the system works in disconnected mode

Over the whole length of each cycle, the two columns must be synchronized regarding the respective flow rates, which are always adjusted by the system software in order for the tasks occurring at the same time at the two columns to have the same duration (e.g. task 2 has the same duration as task 6).

In case only fractions of type Cs or Cw are present, the method of the invention can be adapted by omitting the specific tasks.

No explicit washing step is required in this embodiment of the method.

In this embodiment of the method of the invention, the loading step of steps d) as described above, is performed by loading first the fractions Cw, then the amount b of solution A and finally the fractions Cs.

After the last complete cycle, column 1 undergoes a final gradient elution, wherein the fractions B are collected one last time, resulting from the final loading of fractions C without any further amount of feed solution. This step referred to as "shutdown" is identical to a switch with the only absence of any loading of feed solution.

After the purification of the crude icatibant by the method of the present invention, the pooled fractions B may undergo a subsequent process step meant to exchange the salt counter-ion resulting from the aqueous buffer used during chromatography, for instance triethylammonium, to acetate, which is the salt form commercially used. Such a step is conveniently performed by another HPLC process using a mobile phase comprising for instance an ammonium acetate buffer. The solution collected from this step is then finally filtered and lyophilized to obtain the final API icatibant acetate.

In a preferred embodiment of the method of the invention, the icatibant specification is 99%; the mobile phase comprises a TEAP buffer at 20 mM concentration with acetonitrile as the organic modifier; a C18 bonded silica as stationary phase.

If this preferred embodiment is performed with one column, the fractions $C_W$ and Cs are pooled and loaded onto the chromatographic column before loading the amount b of solution A.

If this preferred embodiment is performed with two identical columns, the loading step is performed by loading first the fractions Cw, then the amount **b** of solution A and finally the fractions Cs.

The method of the present invention allows the preparation of icatibant in pure form and with a very high yield, which is particularly well suited for the industrial application, because surprisingly the very good recovery rate is not affected by the number of cycles that have to be performed to have also larger amounts of icatibant purified. Thereby it is not necessary to invest in larger chromatographic columns, for purifying larger amounts of icatibant. Also, the productivity of the whole process is highly increased and the solvent consumption is remarkably reduced when compared to otherwise necessary single HPLC chromatographic procedures that are standard for purifying peptides for pharmaceutical use. This will be illustrated by the following Examples.

## Abbreviations

| | |
|---|---|
| ACN | Acetonitrile |
| DCM | Dichloromethane |
| DMF | Dimethylformamide |
| CV | Column Volume |
| MP | Mobile Phase |
| SPPS | Solid phase peptide synthesis |
| TFA | Trifluoroacetic acid |
| DIPEA | Diisopropylethylamine |
| DIC | N,N'-diisopropylcarbodiimide |
| OxymaPure | Ethyl-2-cyano-2-hydroxyiminoacetate |
| TEAP | Triethylammonium phosphate |
| (RP)-HPLC | (Reversed Phase)-High performance liquid chromatography |
| RRT | Relative retention time |
| RT | Room temperature |
| T | Temperature |

## Examples

The following examples provide detailed experimental conditions for the method of present invention and are intended to be illustrative and not limiting of all possible embodiments of the same.

Unless indicated otherwise, the composition of liquid mixtures, e.g. mobile phases, are defined as volume % at ambient temperature.

Unless noted otherwise, all materials, solvents and reagents were obtained from commercial suppliers, of the best grade, and used without further purification.

### **Example** 1 - **Preparation of crude icatibant**

Icatibant was prepared by SPPS *via* loading 2-chloro trityl chloride resin with Fmoc-L-Arg(Pbf)-OH by DCM/DIPEA activation as a first step. The resin was then capped at any residual Cl- or OH- free group by a mixture of methanol and acetic anhydride, and the synthesis was continued stepwise by using 2 equivalents of each required

sidechain protected N-Fmoc amino acid (Fmoc-L-Oic-OH, Fmoc-D-Tic-OH, Fmoc-L-Ser(tBu)-OH, Fmoc-L-Thi-OH, Fmoc-Gly-OH, Fmoc-L-Hyp(tBu)-OH, Fmoc-L-Pro-OH, Fmoc-D-Arg(Pbf)-OH), activated with DIC/OxymaPure. Fmoc deprotection was performed by 20% piperidine in DMF. After loading the last residue, Fmoc-D-Arg(Pbf)-OH, Fmoc was removed by the piperidine solution and the resin washed and dried with DCM. A mixture of TFA, triisopropylsilane and 2,2'-(ethylenedioxy)diethanethiol was used to cleave icatibant from the resin. Finally, after resin filtration, cold diisopropylether was added to the solution to precipitate the peptide. After filtration, the solid was dried under vacuum, yielding a crude peptide powder, with purity 88% (HPLC).

The concentration of icatibant in the crude powder was 49% by weight, as determined by dissolving 1 mg sample in 1 mL of ACN:ammonium acetate 50 mM 3:97 buffer solution and by analysing the sample through HPLC (see analytical method, Example 2) and a calibration curve, giving the mg/mL concentration.

**Example 2 - Analytical method**

Every fraction and feed solution have been analysed in HPLC on an Agilent 1290 (Agilent, Santa Clara, CA, USA) according to the following parameters:
MP-A: 0.1% TFA in water
MP-B: 0.1% TFA in acetonitrile
Column: Kromasil 5-100 C18, 250x4.6 mm, 5 micron
Volume of injection: 2 microL
Wavelength: 226 nm
T: 50 °C
Flow rate: 1 mL/min
Gradient slope:

| Time (min) | MP-A (%) | MP-B (%) |
|---|---|---|
| 0 | 80 | 20 |
| 33 | 50 | 50 |
| 36 | 16 | 84 |
| 40 | 16 | 84 |
| 42 | 80 | 20 |
| 50 | 80 | 20 |

The recovery of a single fraction or of pooled fractions was calculated by comparing the obtained HPLC chromatogram with a calibration curve, thus determining the sample concentration and then the absolute quantity of icatibant in the fraction(s). Calibration was performed using samples with known concentration of pure icatibant, ranging from 0.1 to 2 g/L.

**Example 3** - **Preparation of the feed solution (Solution A)**

The feed solution was prepared by dissolving 1g of crude icatibant obtained as described in Example 1 in 200 mL of a solution ACN : ammonium acetate 50 mM 3:97, in order to reach a final icatibant concentration of about 2.5 mg/mL. The pH of the solution was adjusted to about 6.8 with $NH_4OH$ 10%. After one hour stirring, the feed solution was filtered and used as such in the Examples 4, 5 and 6.

**Example 4** - **batch run (method design and comparative example)**

MP-A: TEAP 20 mM : ACN 90 : 10
MP-B: TEAP 20 mM : ACN 50 : 50
Column: Daisogel-SP-120-Ods-BIO, 10 micron, 250x4.6 mm (CV=4.2 mL)
Wavelengths: 280 and 265 nm

The column was equilibrated with 2 CV mobile phase at 12% MP-B, 4 mL/min flow rate. 16.8 mL feed solution (4CV, corresponding to 42 mg icatibant (84 mg crude), calculated as 10 mg per CV(mL)) were loaded at 3 mL/min flow rate.

After the loading, the column was washed with 2 CV at 12% MP-B (4 mL/min flow rate) before gradient start. During the gradient elution (see table below), the percentage of MP-B increased from 12 to 37% during 18 CV, at 1.5

mL/min flow rate. One fraction was collected every 60 seconds (fraction volume 1.5 mL). Finally, the stationary phase was stripped with 100% MP-B for 7 CV (at 4 mL/min flow rate). The total operation time was 67 minutes.

Fractions 26-28 containing icatibant with purity above specification (99 %) were pooled. Purity, recovery and final quantity of icatibant were determined as described in Example 2.

| STEP | Volume (CV) | Flow Rate (mL/min) | MP-A (%) | MP-B (%) |
|---|---|---|---|---|
| Equilibrating | 2 | 4 | 88 | 12 |
| Loading of Solution A | 4 | 3 | 88 | 12 |
| Washing | 2 | 4 | 88 | 12 |
| Gradient | 18 | 1.5 | 88 to 63 | 12 to 37 |
| Stripping | 7 | 4 | 0 | 100 |

Solution A employed: 16,8 mL, corresponding to 84 mg crude icatibant (i.e. 42 mg icatibant).

Final purity: 99.42%; icatibant obtained: 5.2 mg; recovery: 12.41%.

Total mobile phase volume: 137.1 mL

**Example 5 - Reversed phase HPLC purification according to the method of the invention using one column (n = 5)**

All the parameters and operating conditions (mobile phases, column, wavelength) were the same as in Example 4.

The column was equilibrated with 2 CV mobile phase at 12% MP-B, 4 mL/min flow rate. 16.8 mL feed solution (4CV, corresponding to 42 mg icatibant (84 mg crude), calculated as 10 mg per CVmL) were loaded at 3 mL/min flow rate, and the first purification run was performed, comprising washing, gradient elution and stripping steps as described in Example 4. Fractions 19-25 and 29-30 were chosen as recycling fractions (fractions C) containing weak impurities + icatibant (Cw) and strong impurities + icatibant (Cs), respectively, while fractions 26-28 containing icatibant with purity above specification (99%, fractions B) were pooled and put aside. The duration of this run (step b) was 67 minutes.

The column was subjected to a new equilibrating step and fractions C were diluted 1:1 with TEAP 20 mM (10.5 mL x 2 = 21 mL, i.e. 5 CV) and loaded onto the column for the next run (second run). Recovery for this pool was determined and was approximately 50% of the quantity of icatibant loaded (i.e. about 21 mg). Therefore, another 2 CV of feed solution were loaded into the column, corresponding to the remaining 50% of the quantity of icatibant, calculated relatively to the first run. Subsequent steps (washing, gradient elution and stripping) were performed as in the first run (see also the Table below). The single run time was 72 minutes (step d)).

At the end of the second purification run, fractions 21-25 and 29-30 were chosen as recycling fractions Cw and $C_s$, respectively. Fractions 26-28 containing icatibant with purity above 99% were also pooled. The pooled recycling fractions were diluted 1:1 with TEAP 20 mM.

The pooled recycling fractions were subjected to a subsequent run using the same procedure as described above. The method was repeated four times (1+5 total runs). From the second run on, the steady-state was reached, meaning that the UV profiles did not change run after run and the purity and recovery values of the collected pools were constant.

| STEP | Volume (CV) | Flow Rate (mL/min) | MP-A (%) | MP-B (%) |
|---|---|---|---|---|
| Equilibrating | 2 | 4 | 88 | 12 |
| Loading of fractions C | 5 | 3 | 88 | 12 |
| Loading of solution A | 2 | 3 | 88 | 12 |
| Washing | 2 | 4 | 88 | 12 |
| Gradient | 18 | 1.5 | From 88 to 63 | From 12 to 37 |
| Stripping | 7 | 4 | 0 | 100 |

Solution A employed: 58.8 mL, corresponding to 303 mg crude icatibant (i.e. 148.4 mg icatibant)
Final purity: 99.01%; icatibant obtained: 124.4 mg; total recovery 83.82%
Total mobile phase: 887.4 mL; operation time: 427 minutes (7.12 h).

**Example 6 - Reversed phase HPLC purification according to the method of the invention using Two-column Countercurrent Solvent Gradient Purification (n = 10)**

This specific embodiment of the invention was performed by using a ContiChrom® CUBE Combined (ChromaCon/YMC, Zurich, CH) instrument, with software ChromIQ® Version 7, comprising two columns Daisogel-SP-120-Ods-BIO, 10 micron, 150x4.6 mm (same stationary phase as in Example 4, different column size); CV(one column) = 2.5 mL, total column volume = 5 mL(2CV)). Mobile phases were also the same as for Example 4; MP-A was also used as inline dilution phase during the loading of Cw and of Cs fractions.

The connected columns were equilibrated with 2CV mobile phase at 12% MP-B, 4 mL/min flow rate. 10 mL feed solution (4CV, corresponding to 25 mg icatibant (51 mg crude), calculated as 10 mg per CV) were loaded at 3 mL/min flow rate.

The batch run described in Example 4 was taken as reference to establish the set of switching times ($t_A$ to $t_E$), depending on the analysis of the fractions collected. After the switching times were determined, the software calculated automatically the flow rates for each step, in order that the maximum flow rate reached inside the columns during the gradient step was identical to the flow rate of the gradient step in the batch run as described in Example 4 (1.5 mL/min). The amount b of solution A was calculated by considering the quantity of icatibant leaving the system during the collection of fractions B. In this example, an amount corresponding to 67% of the quantity of icatibant loaded at the beginning of the process in column 1 (step b) was collected in fractions B during step b) and at each subsequent step d) (or column switch), therefore 2.7 CV (=67% of 4 CV, 6.7 mL) of solution A were loaded at each step d) together with the recycling fractions. The elution during each column switch was carried out according to the table below and fractions B containing icatibant with purity above 99% were pooled.

| STEP | Volume (CV) | MP-A (%) | MP-B (%) |
|---|---|---|---|
| Equilibrating | 2 | 88 | 12 |
| Loading of fractions $C_w$ | 2,09 | 88 | 12 |
| Loading of solution A | 2,7 | 88 | 12 |
| Loading of fractions $C_s$ | 2,13 | 88 | 12 |
| Gradient | 18 | From 88 to 63 | From 12 to 37 |
| Stripping | 7 | 0 | 100 |

The duration of each cycle was 51 minutes; 5 complete cycles through the system were performed, corresponding to 10 steps d) (n=10). The total operation time, comprising step b) and final shutdown was 286 minutes (4.78 h).

The switching times t (min) determined based on the batch run in Example 4 were:

| tA | tB | tC | tD | tE |
|---|---|---|---|---|
| 6.2 | 19.7 | 21.2 | 24.2 | 25.7 |

All equilibrating and stripping protocols were set according to the batch run of Example 4. From the second cycle on, the steady-state was reached, meaning that the UV profiles did not change cycle by cycle and the values of purity and recovery of the collected pools were constant.

Solution A employed: 77 mL, corresponding to 397 mg crude icatibant (i.e. 194.61 mg icatibant)
Final purity: 99.3%; icatibant obtained: 163.41 mg; total Recovery: 83.97%
Total mobile phase: 804.7 mL

**Summary of the performance of the three Examples 4, 5, 6 (entire process)**

| Example | purity % | recovery % | productivity (mg/mL/h) | solvent consumption (L/g) |
|---|---|---|---|---|
| **4** | 99.42 | 12.41 | 1.11 | 25.64 |
| **5** | 99.01 | 83.82 | 4.16 | 7.13 |
| **6** | 99.30 | 83.97 | 6.83 | 4.92 |

Parameter values increase relative to batch method (Example 4)

| Example | recovery % | productivity (mg/mL/h) | solvent consumption (L/g) |
|---|---|---|---|
| **5** | +575% | +274% | -72% |
| **6** | +577% | +515% | -81% |

## Claims

1. A method for the preparation of icatibant comprising a chromatographic process for removing impurities from a solution A containing icatibant with a purity below specification, comprising the steps of:

   a) providing n +1 amounts of the solution A;
   b) subjecting an amount **a** of solution A to a first reversed phase HPLC purification run, wherein the stationary phase is hydrocarbon bonded silica, the mobile phase comprises an aqueous buffer and an organic modifier, and a gradient elution is effected by gradually increasing the organic modifier concentration within the mobile phase over time; said reversed phase HPLC purification run comprising loading a chromatographic column with the amount **a** of solution A; and wherein one or more fractions B containing icatibant with a purity above specification and one or more fractions C containing icatibant with a purity below specification are collected;
   c) pooling the fractions C;
   d) subjecting the pooled fractions C obtained in step c) together with an amount b of solution A to a further reversed phase HPLC purification run as described in step b); said further reversed phase HPLC purification run comprising loading a chromatographic column with said pooled fractions C and with the amount **b** of solution A;
   e) optionally repeating step c) and d) until the n amounts **b** of solution A have been subjected to at least one reversed phase HPLC purification run as described in step d); and
   f) pooling all fractions B;

   wherein n is at least 1.

2. The method according to claim 1, wherein the stationary phase is selected from the group consisting of C18, C8, C4, phenyl-hexyl, biphenyl, fluorophenyl and phenyl bonded silica, and preferably is C18 bonded silica.

3. The method according to any one of the previous claims, wherein the organic modifier is selected from the group consisting of acetonitrile, methanol, ethanol and tetrahydrofuran, preferably being acetonitrile.

4. The method according to any one of the previous claims, wherein the aqueous buffer comprises an acid, selected from the group consisting of phosphoric acid, hydrochloric acid, trifluoroacetic acid, acetic acid, methanesulphonic acid, formic acid and citric acid; and/or a base, selected from the group consisting of ammonia, triethylamine, diethylamine, dibutylamine, diisopropylethylamine, sodium hydroxide and potassium hydroxide; and/or a salt, selected from the group consisting of formates, like sodium, ammonium and potassium formate; acetates, like sodium, ammonium and potassium acetate; phosphates, like sodium, potassium and triethylammonium phosphate; chlorides, like sodium and ammonium chloride; ammonium carbonate and ammonium bicarbonate; preferably triethylammonium phosphate.

5. The method according to any one of previous claims, wherein the aqueous buffer has a concentration from 0.05 mM to 1 M, preferably from 0.1 to 250 mM, more preferably from 10 to 100 mM, most preferably from 20 to 50 mM.

6. The method according to any one of previous claims, wherein the reversed phase HPLC purification run of at least one of step d) is performed by either loading the chromatographic column with the fractions C and the amount **b** of solution A simultaneously, or consecutively.

7. The method according to any one of previous claims, wherein the reversed phase HPLC purification runs of step b) and steps d) are performed with one chromatographic column.

8. The method according to any one of claims 1 to 6, wherein the reversed phase HPLC purification runs of step b) and steps d) are performed with a system of two or more identical interconnected chromatographic columns, which

can be connected to allow eluting fractions C to be subjected to another reversed phase HPLC purification run of step d); or disconnected to allow the eluate to be discarded or fractions B to be collected.

**9.** The method according to claim 8, which is performed with a system of two identical interconnected chromatographic columns, preferably being an automatedly operating method.

**10.** The method according to any one of claims 1 to 7, wherein the reversed phase HPLC purification run of any step d) is performed on the same chromatographic column as the purification run of step b), after said column has been subjected to a stripping step and an equilibrating step between step b) and step d), and optionally before any subsequent step d).

**11.** The method according to any one of previous claims, wherein the one or more fractions C collected during step b) or during one or more of steps d) are either fractions Cw, comprising icatibant and weak impurities, or fractions $C_s$, comprising icatibant and strong impurities.

**12.** The method according to claim 11, wherein in the reversed phase HPLC purification run of at least one of step d) either the fractions $C_W$ and Cs are pooled and loaded onto the chromatographic column before or after loading the amount **b** of solution A; or the loading step is performed by loading first the fractions Cw, then the amount **b** of solution A and finally the fractions Cs.

**13.** The method according to any of previous claims, further comprising a shutdown step wherein the fractions B are collected one last time, resulting from the final loading of fractions C without any further amount of solution A.

**14.** The method according to any of previous claims, wherein the specification is preferably 95, 95.5, 96, 96.5, 97, 97.5, 98, 98.5, 99 or 99.5%, more preferably 98.5%, and most preferably 99 %.

**15.** The method according to any of previous claims, wherein the quantity of icatibant in the amount b of solution A of step d) is not higher than the quantity of icatibant in the fractions B obtained in step b).

Fig. 1

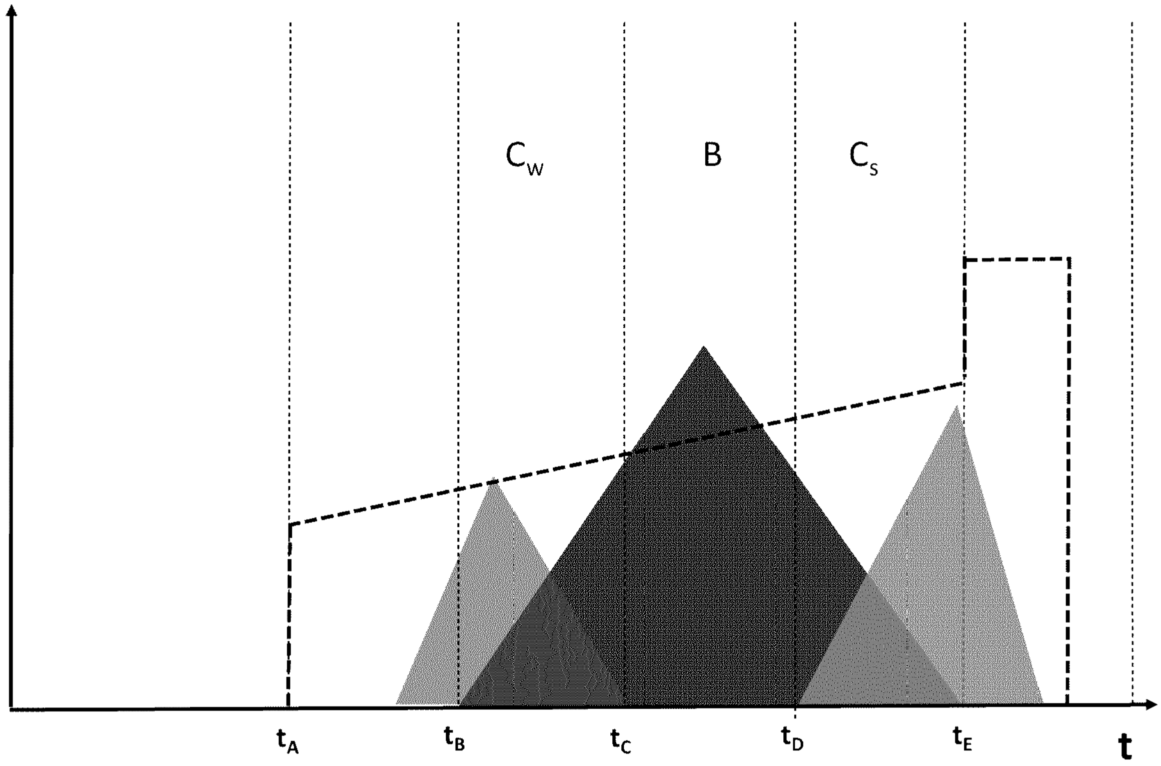
$C_W$
B
$C_S$
$t_A$
$t_B$
$t_C$
$t_D$
$t_E$
t

Fig. 2

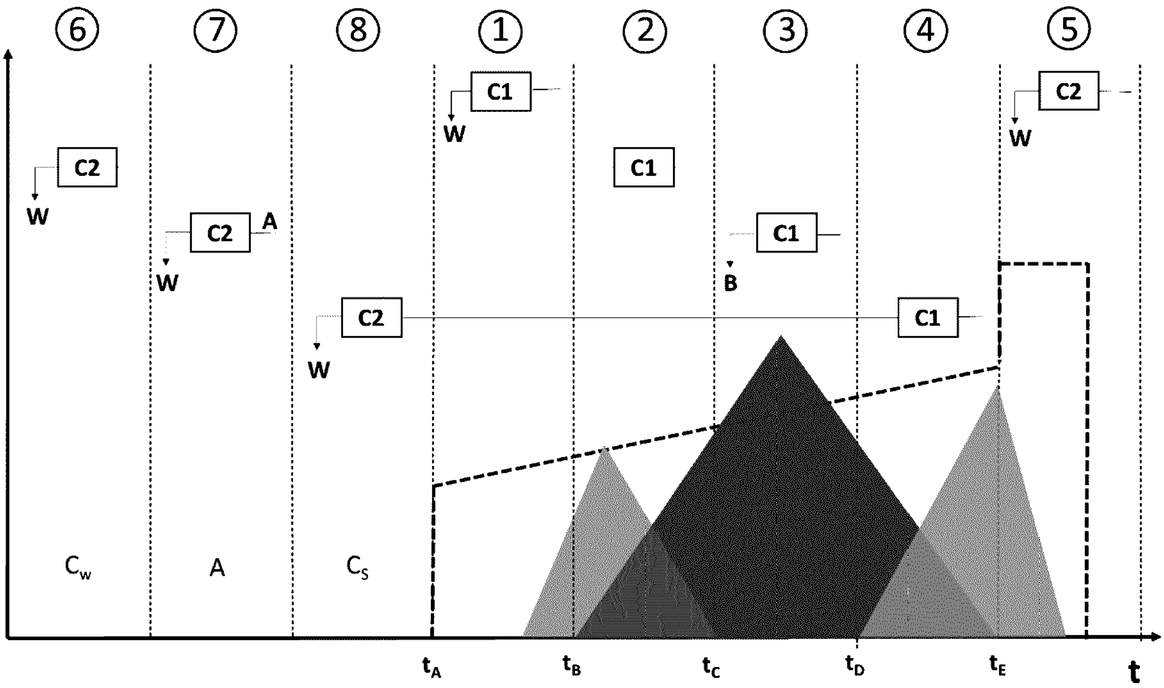
⑥
⑦
⑧
①
②
③
④
⑤
C1
W
C2
C1
C2
W
C2
W
A
C1
B
C2
C1
W
$C_w$
A
$C_S$
$t_A$
$t_B$
$t_C$
$t_D$
$t_E$
t

Fig. 3

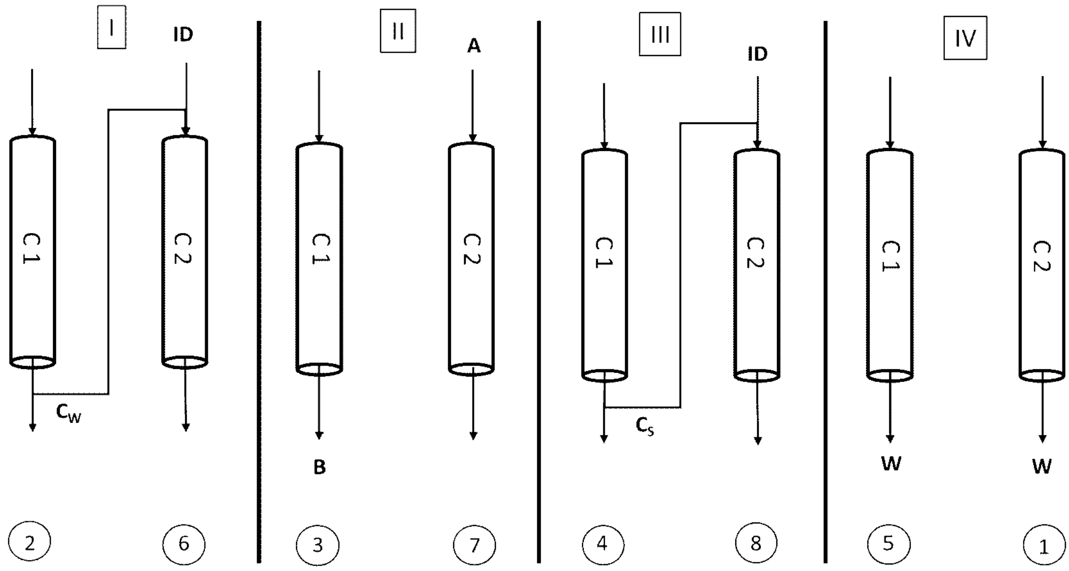

European Patent Office (Europäisches Patentamt / Office européen des brevets)

## EUROPEAN SEARCH REPORT

Application Number
EP 21 17 1717

**DOCUMENTS CONSIDERED TO BE RELEVANT**

| Category | Citation of document with indication, where appropriate, of relevant passages | Relevant to claim | CLASSIFICATION OF THE APPLICATION (IPC) |
|---|---|---|---|
| T | DE LUCA CHIARA ET AL: "Process Intensification for the Purification of Peptidomimetics: The Case of Icatibant through Multicolumn Countercurrent Solvent Gradient Purification (MCSGP)", INDUSTRIAL & ENGINEERING CHEMISTRY RESEARCH, vol. 60, no. 18, 3 May 2021 (2021-05-03), pages 6826-6834, XP55850549, ISSN: 0888-5885, DOI: 10.1021/acs.iecr.1c00520 ----- | | INV. C07K7/06 C07K1/14 B01D15/18 |
| X | STEWART J M ET AL: "A NEW GENERATION OF BRADYKININ ANTAGONISTS", IMMUNOPHARMACOLOGY, ELSEVIER SCIENCE PUBLISHERS BV, XX, vol. 33, no. 1/03, 1 January 1996 (1996-01-01), pages 51-60, XP000610702, ISSN: 0162-3109, DOI: 10.1016/0162-3109(96)00084-7 | 1 | |
| Y | * abstract; section 2 * ----- | 2-15 | TECHNICAL FIELDS SEARCHED (IPC) |
| Y | CN 104 072 585 A (CHENGDU SHENGNUO BIOTEC CO LTD) 1 October 2014 (2014-10-01) * Embodiment 24 * ----- | 1-15 | C07K B01D |
| Y | US 2020/331960 A1 (ROSE MICHAEL HARRY [GB]) 22 October 2020 (2020-10-22) * par. 9 * ----- | 1-15 | |
| Y | US 5 935 932 A (STEWART JOHN M [US] ET AL) 10 August 1999 (1999-08-10) * col. 6 * ----- | 1-15 | |
| Y | US 2020/355655 A1 (AUMANN LARS [CH] ET AL) 12 November 2020 (2020-11-12) * abstract; Fig. 1 * ----- | 1-15 | |

1

The present search report has been drawn up for all claims

| Place of search | Date of completion of the search | Examiner |
|---|---|---|
| Munich | 13 October 2021 | Hohwy, Morten |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 21 17 1717

This annex lists the patent family members relating to the patent documents cited in the above-mentioned European search report. The members are as contained in the European Patent Office EDP file on
The European Patent Office is in no way liable for these particulars which are merely given for the purpose of information.

13-10-2021

| Patent document cited in search report | Publication date | Patent family member(s) | Publication date |
|---|---|---|---|
| CN 104072585 A | 01-10-2014 | NONE | |
| US 2020331960 A1 | 22-10-2020 | AU 2018304867 A1 | 02-01-2020 |
| | | BR 112019027760 A2 | 07-07-2020 |
| | | CA 3067496 A1 | 24-01-2019 |
| | | CL 2020000087 A1 | 24-07-2020 |
| | | CN 110945008 A | 31-03-2020 |
| | | CO 2020000090 A2 | 24-04-2020 |
| | | EA 202090262 A1 | 12-05-2020 |
| | | EP 3655420 A1 | 27-05-2020 |
| | | JP 2020527553 A | 10-09-2020 |
| | | KR 20200027552 A | 12-03-2020 |
| | | SG 11201911880U A | 30-01-2020 |
| | | US 2020331960 A1 | 22-10-2020 |
| | | WO 2019016154 A1 | 24-01-2019 |
| US 5935932 A | 10-08-1999 | NONE | |
| US 2020355655 A1 | 12-11-2020 | CA 3084473 A1 | 23-05-2019 |
| | | CN 111356512 A | 30-06-2020 |
| | | DK 3512616 T3 | 03-02-2020 |
| | | EP 3512616 A1 | 24-07-2019 |
| | | ES 2766200 T3 | 12-06-2020 |
| | | HU E047746 T2 | 28-05-2020 |
| | | JP 2021503367 A | 12-02-2021 |
| | | KR 20200088825 A | 23-07-2020 |
| | | PL 3512616 T3 | 01-06-2020 |
| | | PT 3512616 T | 29-01-2020 |
| | | SG 11202003440S A | 28-05-2020 |
| | | SI 3512616 T1 | 31-03-2020 |
| | | US 2020355655 A1 | 12-11-2020 |
| | | WO 2019096622 A1 | 23-05-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 104072585
- WO 2019202057 A


### Non-patent literature cited in the description


- *CHEMICAL ABSTRACTS,* 130308-48-4
- **DE LUCA et al.** *Trends in Analytical Chemistry,* 2020, vol. 132, 116051